# DEMANDE DE BREVET EUROPEEN

(11) **EP 4 575 490 A1**
(43) Date de publication de la demande: **25.06.2025**
(21) Numéro de dépôt: 23217908.5
(22) Date de dépôt: 19.12.2023
(51) Int. Cl.: G01N 33/00, G01N 33/497

(54) **DISPOSITIF ET MÉTHODE D'ANALYSE DE BIOMARQUEURS GAZEUX**

(71) Demandeur: Zynnon AG, 8834 Schindellegi (CH)
(72) Inventeur: Abousaleh, Khaled, 8834 Schindellegi (CH)
(74) Mandataire: P&TS SA (AG, Ltd.)

(57) **Abrégé**

La présente invention porte sur un dispositif de détection (10) adapté à l'analyse de l'air ambiant d'une pièce comprenant plusieurs capteurs et où les paramètres de détections sont contrôlées indépendamment pour chaque capteurs. L'invention porte en outre sur un système d'analyse des données collectées par un tel dispositif et sur une méthode de détection et d'analyse de telles données.

## Description

### Domaine technique

La présente invention concerne un dispositif et un système de détection et d'analyse de biomarqueurs gazeux. Le dispositif objet de la présente invention permet en particulier de détecter la présence d'un ou plusieurs biomarqueurs gazeux et d'établir un profil des biomarqueurs gazeux détectés, ainsi que de suivre l'évolution d'un tel profil dans le temps. La présente invention concerne en outre une méthode de diagnostique permettant d'identifier une pathologie de manière non invasive, sur la base des biomarqueurs identifiés. La présente méthode permet en outre de suivre l'évolution d'une pathologie sur la base des biomarqueurs identifiés et d'adapter le traitement en conséquence.

### Etat de la technique

Des dispositifs permettent de mesurer ponctuellement des biomarqueurs émis par la respiration d'un patient et de déterminer à des intervalles de temps donnés la présence de tels biomarqueurs. Ces dispositifs requièrent néanmoins un contact physique avec le patient, qui doit en général souffler dans un collecteur de sorte que son haleine soit analysée. Les biomarqueurs recherchés ne sont cependant pas détectables de manière homogène à chacune des expirations du patient. Leur concentration peut même varier significativement au cours du temps sans nécessairement être corrélée à l'état physiologique du patient. Les analyses ponctuelles sont donc sujettes à des erreurs d'interprétation ou nécessitent de répéter fréquemment les mesures, ce qui peut être contraignant.

Les biomarqueurs ciblés peuvent permettre, par exemple, de déterminer le taux de glucose dans le sang d'un patient, comme décrit dans le document WO2022233771.

Des dispositifs tels que celui décrit dans le document EP4244617 permettent de surveiller en continu un espace fermé tel qu'un bureau ou une salle classe, de sorte à identifier la présence de potentiels contaminants. Même utilisés dans le milieu hospitalier, de tels dispositifs ne permettent pas de diagnostiques précis et se limitent à la détection de biomarqueurs spécifiques.

Le document US2023127176 décrit un dispositif comportant un ventilateur et un capteur de biomarqueurs gazeux, couplé à un spectromètre de masse, permettant d'identifier d'éventuels contaminants ou pathogènes susceptibles de propager des épidémies. La surveillance des lieux ne représente pas un diagnostique ou un suivi thérapeutique d'un patient. L'identification individuelle des biomarqueurs gazeux ne permet pas nécessairement de poser un diagnostique précis et fiable.

L'analyse des biomarqueurs gazeux n'est à ce jour pas totalement intégrée dans les processus thérapeutiques. Il y a matière à davantage exploiter les biomarqueurs gazeux à des fins de diagnostique, de suivi thérapeutique et de recherche.

### Bref résumé de l'invention

Un but de la présente invention est de proposer un dispositif et/ou un système amélioré permettant notamment de suivre dans le temps, de manière continue, le profil de biomarqueurs gazeux d'un patient, de préférence sans contact physique avec le patient. Un autre but de la présente invention est de proposer un dispositif et/ou un système permettant de suivre l'état de santé d'un patient sous traitement et d'en déduire la pertinence du traitement.

Un autre but de l'invention est de proposer une méthode permettant de suivre dans le temps, de manière continue, le profil de biomarqueurs gazeux d'un patient, de préférence sans contact physique avec le patient, de sorte à établir un diagnostique. Un autre but de la présente invention est de proposer une méthode d'évaluation d'un traitement d'un patient, en particulier pour éviter les traitements non adaptés et favoriser les traitements les plus adéquats.

Un autre but de la présente invention est de proposer un système et/ou une méthode permettant de suivre aisément et à distance l'état de santé d'un patient sous traitement et d'évaluer la pertinence de son traitement. Le suivi à distance permet notamment une prise en charge ambulatoire des patients plus efficace et moins onéreuse.

Un autre objectif de la présente invention est de proposer un système et/ou une méthode permettant de limiter les effets néfastes des traitements, en particulier des antibiotiques. L'objectif est de limiter la résistance bactérienne.

Un autre objectif de la présente invention est de proposer un système et/ou une méthode permettant d'identifier une pathologie et de sélectionner et/ou de simuler un traitement thérapeutique.

Selon l'invention, ces buts sont atteints notamment au moyen du système et de la méthode objet des revendications indépendantes et détaillées dans les revendications dépendantes.

Cette solution présente notamment l'avantage par rapport à l'art antérieur de proposer des solutions thérapeutiques plus rapides, plus efficaces et plus confortables. La présente solution permet en outre de minimiser les coûts d'analyse à des fins de diagnostique.

### Brève description des figures

Des exemples de mise en oeuvre de l'invention sont indiqués dans la description illustrée par les figures suivantes :
- Figure 1 : Représentation schématique des éléments de détection du dispositif de détection selon un mode de réalisation de la présente invention,
- Figure 2 : Représentation schématique d'un dispositif de détection selon un mode de réalisation de la présente invention,
- Figure 3 : Représentation schématique du système d'analyse selon un mode de réalisation de la présente invention.

### Exemple(s) de mode de réalisation de l'invention

La présente invention concerne un dispositif de détection **10** de composés volatiles, en particulier de composés organiques volatiles également connus sous l'acronyme anglais VOC (volatile organic compounds). Le dispositif de détection **10** permet de détecter une grande variété de composés volatiles présents dans l'atmosphère dans lequel il est placé. Le dispositif de détection **10** peut être calibré pour détecter plus précisément certains composés volatiles. En particulier, le dispositif de détection **10** peut être destiné à détecter les produits volatiles présents dans un intérieur, tel qu'une habitation, une chambre d'hôpital, un bâtiment privé ou publique ou tout aire close. Le présent dispositif de détection **10** est particulièrement adapté à la détection des produits volatiles émis par des systèmes biologiques, en l'occurrence un ou plusieurs patients, dans une pièce. A cet effet, le dispositif de détection **10** est placé à proximité de la personne surveillée et analyse un ou plusieurs échantillons d'air susceptibles de contenir les produits organiques émis par la personne présente dans la pièce. Typiquement, le dispositif d'analyse **10** se présente sous la forme d'un boitier comprenant des ouvertures sur au moins une de ses faces de sorte à laisser l'air ambiant cheminer jusqu'aux éléments de détection disposés au sein du boitier. Le dispositif de détection n'a pas dans ce cas vocation à collecter précisément et exclusivement l'air expiré par un patient. Il est donc dénué de masque ou de collecteur habituellement disposé sur la bouche d'un patient pour en collecter l'haleine. Il en résulte que le dispositif de détection **10** selon la présente invention collecte tous les composés volatiles présents dans la pièce indépendamment de leur origine. Le ou les échantillons d'air correspondent ici à un aliquot de l'atmosphère.

Selon un autre mode de réalisation, un échantillon d'air dénote un volume d'air expiré par un patient, prélevé au niveau de son visage et acheminé jusqu'au dispositif de détection **10**. Cette disposition peut être utile lors d'une détection urgente pour un diagnostique rapide, par exemple en pharmacie ou lors de l'intégration d'un patient en milieu hospitalier. Cette disposition n'exclue pas qu'une surveillance peut être ensuite établie au moyen du dispositif de détection ici-décrit sans contact avec le patient. Le même dispositif de détection **10** peut ainsi être utilisés sous différentes configurations.

Selon un autre mode de réalisation, le dispositif de détection **10** selon la présente description peut être intégré à un automate d'analyse en laboratoire. Par exemple, des échantillons de cultures cellulaires peuvent être confinés dans une enceinte dont l'air est échantillonné et/ou analysé au moyen du dispositif de détection **10** ici-décrit. Selon une disposition particulière, un même dispositif de détection **10** peut être dédié à la surveillance ou l'analyse de plusieurs enceintes distinctes.

Le présent dispositif de détection **10** n'est en outre pas nécessairement pourvu d'un ventilateur concentrant l'air vers les éléments de détection internes, pour autant que l'air puisse parvenir jusqu'aux éléments de détection internes. Cependant, certaines applications peuvent nécessiter un tel ventilateur ou un élément équivalent permettant d'orienter un flux d'air vers les éléments de détection internes.

Selon un mode de réalisation, le dispositif de détection **10** peut être disposé à une hauteur prédéterminée, telle que 80 cm à 1,5 m au-dessus du lit d'un patient. Cette disposition est particulièrement adaptée à la surveillance d'un patient alité, que ce soit en milieu hospitalier ou à domicile. Alternativement, le dispositif de détection **10** peut être placé sur un meuble de la pièce, à une hauteur adaptée pour collecter de manière optimale les composés organiques volatiles. En l'occurrence, une hauteur intermédiaire entre le sol et le plafond est privilégiée de sorte à pouvoir capter au mieux tous les éléments volatiles de la pièce. Une telle disposition est davantage adaptée à la surveillance de personnes mobiles bien que résidant longtemps dans une pièce donnée.

Il est ici précisé que les composés volatiles organiques, ou biomarqueurs gazeux, peuvent être émis par tout type d'organisme vivant et peuvent être détectés dans plusieurs produits sécrétés par un patient, incluant l'urine, la transpiration, le sang l'haleine et tout autre fluide corporel. A ce titre, le dispositif de détection ici-décrit peut être dédié à l'analyse plus spécifique de l'une ou l'autre de ces sécrétions.

Selon un mode de réalisation le dispositif de détection **10** peut être calibré de sorte à détecter uniquement les composés organiques volatiles émis par une personne et pouvant être interprétés comme biomarqueurs. Il peut alternativement permettre la détection d'autres composés volatiles ou en suspension tels que des pollens, des contaminants chimiques ou biologiques, éventuellement présents dans l'espace surveillé. La détection des composés volatiles ou en suspension autres que les potentiels biomarqueurs peut faire l'objet d'un traitement spécifique. Par exemple, bien qu'ils soient physiquement détectés ou détectables par le dispositif de détection 10, les données recueillies peuvent occulter ou ne pas tenir compte de leur présence. Alternativement, les données relatives à des composés volatiles ou en suspension, contaminants, peuvent être analysées conjointement ou séparément des données relatives aux éventuels biomarqueurs de sorte à fournir une analyse plus précise.

Selon un mode de réalisation, le dispositif de détection **10** permet d'établir un profil des composés volatiles présents dans un ou plusieurs échantillons d'air, qu'il s'agisse de l'air ambiant ou d'air collecté à une source particulière telle qu'une enceinte de culture cellulaire ou au niveau du visage d'un patient. Le dispositif de détection **10** comporte au moins un, de préférence plusieurs, capteurs **10a**, **10b**, **10c**, **10n** aptes à produire une réponse numérique en contact d'un ou plusieurs des composés volatiles. Selon la nature des composés volatiles, la réponse d'un capteur donné peut être différente, notamment en termes d'intensité de réponse. L'intensité de réponse d'un capteur ne reflète donc pas nécessairement la quantité d'un composé volatile dans l'air ou sa concentration. En outre, la nature d'un composé volatile peut ne pas être déterminée par un capteur donné.

Le dispositif de détection **10** peut donc ne pas être totalement adapté à l'identification directe de composés volatiles présents dans les échantillons d'air. En particulier un capteur peut ne pas être spécifique d'un composé volatile donné. L'aspect important est qu'en réponse à la détection des composés volatiles, le ou les capteurs produisent un profil représentatif des composés volatiles présents dans l'échantillon d'air.

Selon un mode de réalisation, un ou plusieurs des capteurs **10a**, **10b**, **10c**, **10n** du dispositif de détection **10** peuvent être calibrés au moyen d'un composé volatile connu à des concentrations variables et/ou dans des conditions contrôlées pour en déterminer leur réponse. Alternativement ou en plus, des mélanges connus de composés volatiles identifiés peuvent être utilisés pour la calibration d'un ou plusieurs capteurs **10a**, **10b**, **10c**, **10n**. Les réponse obtenues produisent des profils représentatifs des mélanges analysés.

Un profil désigne ici, par exemple, une réponse électrique d'un ou plusieurs capteurs **10a**, **10b**, **10c**, **10n**. Il peut être matérialisé par une mesure d'intensité électrique, de conductance, de fréquence, de champs magnétique, de longueur d'onde lumineuse ou de toute valeur physique appropriée. Alternativement, un profil peut être matérialisé ou complété par la variation de l'une ou plusieurs de ces valeurs physiques. De telles variations peuvent intervenir notamment lors de changements des conditions de détection, qu'elles soient contrôlées et délibérées ou bien issues de facteurs externes non contrôlés. Typiquement, les conditions de température ou d'humidité peuvent faire varier la réponse d'un ou plusieurs des capteurs du dispositif de détection **10**.

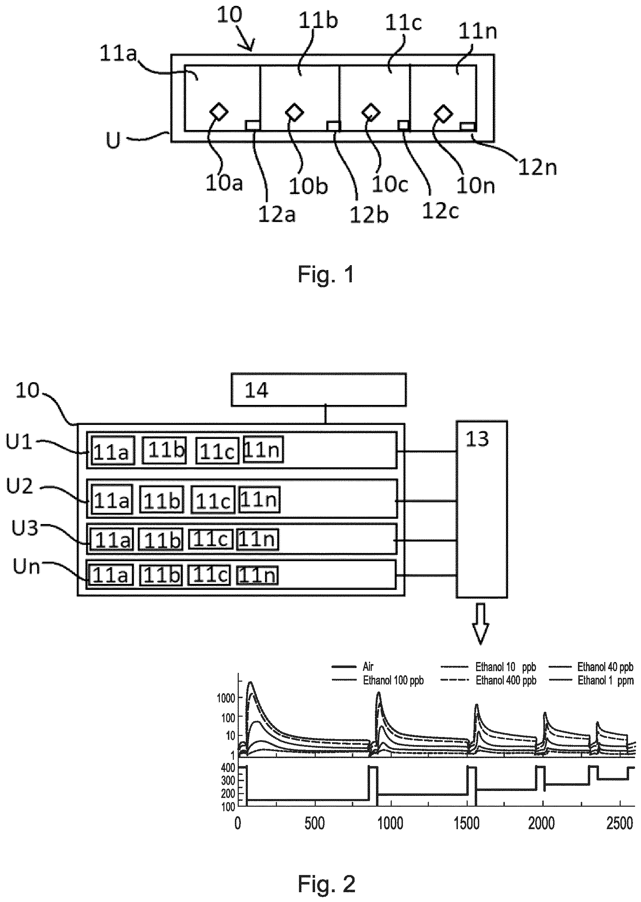

Selon un arrangement particulier, le dispositif de détection **10** selon la présente invention, illustré par la figure 1, comprend des éléments de détection interne à son boitier. De tels éléments de détection comprennent au moins une, de préférence plusieurs, cellules distinctes **11a**, **11b**, **11c**, **11n**. Toutes les cellules sont en contact avec le ou les échantillons d'air. Elles peuvent cependant ne pas être en communication les unes avec les autres, de sorte que l'air traversant une cellule donnée n'est pas analysé par d'autres cellules. Alternativement, les cellules ou une partie des cellules peuvent être arrangées en série de sorte qu'un flux d'air donné puisse traverser plusieurs cellules. Le nombre de cellules n'est pas limitatif. De préférence, le dispositif de détection **10** comprend deux ou plus de deux cellules, notamment 4 ou un multiple de 4.

Chacune des cellules **11a**, **11b**, **11c**, **11n** est pourvue d'au moins un capteur **10a**, **10b**, **10c**, **10n** apte à réagir au contact d'au moins un composé volatile organique. En d'autre termes, un capteur produit un signal électrique mesurable au contact de tels composés volatiles. De tels capteurs peuvent par exemple comprendre des oxydes métalliques ou être du type oxyde métallique comme ceux désignés par l'acronyme MOS (Metal Oxyde sensors). D'autres types de capteurs peuvent être utilisés selon les besoins. En l'occurrence, des capteurs colorimétriques peuvent être utilisés à la place ou en complément des capteurs de type MOS.

Selon un mode de réalisation, une cellule donnée comporte plus d'un capteur. Les capteurs d'une cellule donnée peuvent ainsi être identiques de sorte à produire une mesure plus fiable. Dans ce cas, la réponse des capteurs d'une cellule peut être combinée ou moyennée. Alternativement, les capteurs d'une cellule donnée peuvent être de types différents ou bien s'ils sont de même type, tels que MOS ou colorimétrique, ils peuvent être calibrés pour produire une réponse différente au contact d'un composé volatile donné ou d'un mélange de composition donnée. Ainsi, au sein d'une même cellule, un composé volatile ou un mélange de composés volatiles peut être identifié de manière différente par plusieurs capteurs, ce qui permet de fournir une réponse plus précise, notamment en termes de composition de l'air analysé.

Alternativement ou en plus, les différentes cellules **11a**, **11b**, **11c**, **11n** du dispositif de détection **10** peuvent être pourvues de moyens de contrôle d'au moins un paramètre de mesure. Les paramètres de mesure incluent notamment la température, l'hygrométrie ou la pression. La valeur de l'un ou plusieurs de ces paramètres peut donc être déterminée de sorte que la réponse du ou des capteurs correspondants puisse être modulée en conséquence. Par exemple, la réponse d'un capteur vis-à-vis de certains composés volatile peut varier selon la valeur des paramètres de mesure. Il convient donc de combiner, corriger ou pondérer les données obtenues par les capteurs avec les valeurs des paramètres de mesure ou de certains d'entre eux.

En outre, le contrôle des paramètres de mesure peut impliquer de modifier activement et de manière contrôlée la valeur de l'un ou plusieurs des paramètres de mesure au sein d'une cellule donnée **11a**, **11b**, **11c**, **11n**. Selon un mode de réalisation, une ou plusieurs des cellules du dispositif de détection comporte un dispositif de régulation d'au moins l'un des paramètres de mesure. Typiquement, l'une ou plusieurs des cellules peuvent comprendre un dispositif de régulation thermique **12a**, **12b**, **12c**, **12n** permettant de faire varier de manière contrôlée la température au sein des cellules correspondantes.

Le terme de cellule peut désigner des espaces physiquement séparés au sein du dispositif de détection **10**. Alternativement les cellules peuvent se limiter à des aires de détection pourvues des capteurs correspondants. Dans ce cas, les paramètres de mesure peuvent être contrôlés directement au niveau du capteur **10a**, **10b**, **10c**, **10n** ou à proximité. Par exemple, un ou plusieurs des capteurs peuvent être combinés à un dispositif thermorégulateur de sorte que les gaz présents dans l'air soit détecté indépendamment par chaque capteur à une température prédéterminée.

Selon un mode de réalisation, un ensemble de cellules **11a**, **11b**, **11c**, **11n** forme une unité de détection **U**. Le dispositif de détection **10** peut comprendre plusieurs unités de détection **U1**, **U2**, **U3**, Un comprenant chacune plusieurs cellules telles que celles décrites plus haut (Figure 2). La température de chacune des cellules d'une unité de détection **U** peut être individuellement contrôlée.

Selon un mode de réalisation, le dispositif de détection **10** comprend au moins une unité de détection **U**, de préférence deux unités de détections ou plus, adaptées pour des mesures statiques dans lesquelles les paramètres de mesures ne varient pas. Par exemple, le dispositif de détection **10** peut comporter une première unité de détection **U1** comprenant 4 cellules **11a**, **11b**, **11c**, **11n** de températures respectives **Ta1, Tb1, Tc1 et Tn1.** La valeur des températures peut être fixe et déterminée entre une valeur minimale **Tm1** et une valeur maximale **TM1**. La valeur minimale peut être de l'ordre de 80°C ou 100°C. La valeur maximale peut être de l'ordre de 200°C ou 300°C ou 400°C ou plus selon les besoins. La différence de température entre les cellules d'une unité de mesure **U** peut être une valeur absolue de l'ordre de quelques dizaines de degrés, par exemple 15°C, 20°C ou 25°C ou 30°C ou des multiples ou des combinaisons de ces valeurs. Alternativement, la différence de température entre les cellules d'une unité de détection **U** peut être déterminée de manière relative, par exemple sur la base d'un pourcentage de la température de la cellule voisine. La température peut par exemple être plus grande ou plus petite d'une cellule à la cellule voisine d'une valeur de 5%, 10% ou de 15% ou d'un multiple de ces valeurs.

Selon un exemple, les températures **Ta1**, **Tb1**, **Tc1**, et **Tn1** des cellules de la première unité de détection **U1** sont respectivement de 100°C, 150°C, 175°C et 200°C. De la sorte, l'air est analysé simultanément par les cellules de la première unité de détection **U1** à des températures différentes et les gaz qu'il contient y sont détectés dans des conditions contrôlées distinctes.

Le dispositif de détection **10** peut comprendre une seconde unité de mesure **U2** comprenant 4 cellules **11a**, **11b**, **11c**, **11n** de températures respectives **Ta2**, **Tb2**, **Tc2** et **Tn2.** La valeur des températures peut être fixe et déterminée entre une valeur minimale **Tm2** et une valeur maximale **TM2**. Les valeurs minimale **Tm1** et maximale **TM2** des températures de la seconde unité de détection **U2** peuvent être identiques ou différentes de celles de la première unité de détection **U1**. Les différences de températures entre les cellules de la seconde unité de détection **U2** peuvent être déterminées de manière identique ou différente de la première unité de détection **U1**.

Selon un mode de réalisation, la gamme de températures de la seconde unité de détection **U2** est différente de celle de la première unité de détection **U1.** Par exemple la température minimale **Tm2** de la seconde unité de détection **U2** peut être de l'ordre de 200°C ou de 220°C ou de 250°C. La température maximale **TM2** de la seconde unité de détection **U2** peut être de l'ordre de 400°C ou plus.

Selon un exemple, les températures **Ta2**, **Tb2**, **Tc2**, et **Tn2** des cellules de la seconde unité de détection **U2** sont respectivement de 250°C, 300°C, 350°C et 400°C. De la sorte, les gaz sont détectés par les cellules de la seconde unité de détection **U2** à des températures différentes et dans une gamme de températures différente de celle de la première unité de détection.

Les première **U1** et seconde **U2** unités de détection, telles que décrites ci-dessus fonctionnent en mode dit statique, c'est-à-dire à paramètre de mesure constant. Elles sont adaptées pour des hautes ou relativement hautes concentrations de composés organiques volatiles.

Les paramètres de mesure sont ici limités à la température. Il n'est cependant pas exclus que d'autres paramètres de mesures soient considérés.

Selon un mode de réalisation, une ou plusieurs des unités de détection **U** du dispositif de détection **10** sont adaptées pour des mesures dynamiques, dans lesquelles au moins un des paramètres de mesure varie au cours d'une mesure. Par exemple, le dispositif de détection **10** peut comprendre une troisième unité de détection **U3**, composée de 4 cellules **11a**, **11b**, **11c**, **11n** de températures respectives **Ta3**, **Tb3**, **Tc3** et **Tn3** pouvant varier indépendamment entre une valeur minimale et une valeur maximale. Les valeurs minimale et maximale des cellules d'une unité de détection U peuvent être identiques. Dans ce cas, la variation de température des différentes cellules s'effectue selon des progressions différentes. Alternativement ou en plus, l'une au moins des valeurs minimale et maximale diffère d'une cellule à l'autre au sein de l'unité de détection.

Selon un mode de réalisation, la variation de température au sein d'une cellule peut être linéaire et rectiligne. Alternativement, elle peut être établie selon une variation exponentielle. Alternativement, elles peut être établie par sauts avec des paliers de durée déterminée. Les conditions de variation de température peuvent être adaptées selon les besoins. La variation de température est de préférence négative. Elle peut néanmoins être positive.

Selon un exemple, les températures des cellules **11a**, **11b**, **11c**, **11n** de la troisième unité de détection **U3** varient respectivement de 400°C à 100°C, de 400°C à 150°C, de 400°C à 200°C et de 400°C à 250°C.

Le mode de détection dynamique est favorable à la détection de faibles concentrations de composés volatiles. En outre, une meilleure sélectivité est obtenue. Lors de la variation de température, la relaxation de la conductance, notamment dans le cas où des capteurs de type MOS sont utilisés, est mesurée pour chacun des capteurs **10a**, **10b**, **10c**, **10n** des cellules considérées.

De préférence, la variation de température est rapide, de manière à favoriser la sensibilité. Typiquement, la variation de température intervient dans un intervalle de l'ordre de 1 seconde, ou de quelques centièmes de secondes.

Selon un mode de réalisation, plusieurs cycles de températures sont répétés pour chacune des cellules d'une unité de détection **U**. Les valeurs minimale et maximale ainsi que les conditions de variation des températures sont reproduites à l'identique pour chaque cycle. Alternativement, l'une ou l'autre des valeurs minimale, maximale et de variation changent d'un cycle thermique à l'autre.

Le paramètre de mesure variable est ici la température. Il n'est cependant pas exclus que d'autres paramètres de mesures soient également considérés dans le cadre d'une mesure dynamique, en remplacement ou en complément de la température.

Selon un mode de réalisation, une unité de détection **U** du dispositif de détection **10** est utilisée pour une détection standard des composés volatiles. La détection standard vise à produire un signal global correspondant aux composés volatiles présents dans l'atmosphère. Un tel signal global n'est en soit pas suffisant pour établir le profil des biomarqueurs détectés mais peut être utilisé comme référence dans le traitement des données collectées.

La détection standard peut être effectuée dans des conditions contrôlées. Alternativement, les conditions de mesures correspondent aux conditions ambiantes et ne nécessitent pas de contrôler les paramètres de mesure. Dans ces conditions, l'air est analysé à température ambiante. Les autres paramètres de mesure tels que l'hygrométrie ou la pression peuvent également être ceux de l'environnement. Cette disposition n'exclut pas que l'un ou plusieurs des paramètres de mesure, notamment la température ambiante, soit déterminée au niveau de l'unité de détection correspondante. Selon cet arrangement toutes les cellules de l'unité de détection **U** possèdent les mêmes paramètres de mesure. La nature des capteurs **10a**, **10b**, **10c**, **10n** au sein de ces cellules peut être avantageusement différente d'une cellule à l'autre ou calibrée différemment.

Selon un mode de réalisation, le dispositif de détection **10** est adapté à la détection de composés volatiles simultanément en mode statique et en mode dynamique. Selon un mode de réalisation, le dispositif de détection **10** est adapté à la détection de composés volatiles simultanément en mode statique, en mode dynamique et en mode standard. Selon un mode de réalisation, le dispositif de détection **10** est adapté à la détection de composés volatiles simultanément en mode statique et en mode standard. Selon un mode de réalisation, le dispositif de détection **10** est adapté à la détection de composés volatiles simultanément en mode dynamique et en mode standard. Les combinaisons de modes mentionnées ici représentent des modes hybrides.

Par exemple, une première **U1**, une seconde **U2** et une troisième U3 unités de détection peuvent être utilisées en mode dynamique, selon lequel la température de chacune des cellules d'une unité de détection varie de manière indépendante depuis une température maximale vers une température minimale ou réciproquement. Les gammes de températures et/ou les conditions de variation peuvent varier d'une unité de détection à l'autre. Une quatrième unité de détection **U4** peut être dédiée à la mesure standard des composés volatiles.

Selon un autre exemple, une première **U1**, une seconde **U2** et une troisième **U3** unités de détection peuvent être utilisées en mode dynamique, selon lequel la température de toutes les cellules d'une unité de détection varie de manière simultanée depuis une température maximale vers une température minimale ou réciproquement. Les gammes de températures et/ou les conditions de variation peuvent varier d'une unité de détection à l'autre. Une quatrième unité de détection **U4** peut être dédiée à la mesure standard des composés volatiles.

Selon un autre exemple, une première **U1** et une seconde **U2** unités de détection peuvent être utilisées en mode dynamique, selon lequel la température de toutes les cellules d'une unité de détection varie de manière simultanée depuis une température maximale vers une température minimale ou réciproquement. Une troisième unité de détection **U3** est utilisée en mode statique tel que décrit plus haut. Une quatrième unité de détection **U4** peut être dédiée à la mesure standard des composés volatiles.

Selon un mode de réalisation particulier, le mode est automatiquement adapté en fonction des conditions de mesure. Par exemple, un mode statique peut être initié par défaut pour au moins une unité de mesure **U.** Si le signal de l'une ou plusieurs des cellules de l'unité de mesure correspondante est inférieur à une valeur prédéterminée, l'unité de détection peut être pilotée selon un mode dynamique. Un mode dynamique peut être sélectionné par défaut, où par exemple la température de toutes les cellules de l'unité de détection, ou des unités de détection, varie de manière simultanée depuis une température maximale vers une température minimale ou réciproquement.

Selon un mode de réalisation, les première **U1**, seconde **U2** et troisième **U3** unités de détection sont par défaut en mode statique et la quatrième unité de détection **U4** est en mode standard. Chacune des première **U1**, seconde **U2** et troisième **U3** unités de détection peut automatiquement passer en mode dynamique indépendamment si le signal de l'une ou plusieurs des cellules correspondantes est inférieur à une valeur seuil.

La variété des modes statique, dynamique et hybride, qui ne se limite pas à ceux effectivement décrits ici, permet une grande flexibilité et précision de mesure.

Les modes statiques, dynamiques, standard et hybrides sont décrits ici en combinaison avec des capteurs de type MOS. Cela n'a pas vocation à en limiter l'application aux seuls capteurs de ce type. Selon un mode de réalisation, l'inégalité des cellules d'une ou plusieurs des unités de détection **U**, ou certaines d'entre elles comporte des capteurs autres que ceux de type MOS. Par exemple des capteurs de type colorimétriques ou d'autres types peuvent constituer une unité de détection. Dans ce cas, les conditions de mesures peuvent être adaptées de manière similaires à celles décrites plus haut.

Chacun des capteurs **10a**, **10b**, **10c**, **10n** produit un signal de réponse dans des conditions de mesure contrôlées. Les signaux de réponse sont traités par cellule et par unité de détection de sorte à établir un profil représentatif de la composition de l'air en termes de composés volatiles.

Le dispositif de détection **10** comprend à cet effet une unité de traitement des données **13** collectées pour établir un tel profil. Alternativement, une unité de traitement des données **13** peut être connectée au dispositif de détection **10** de sorte à traiter les données à distance.

Le dispositif de détection **10** comprend en outre une unité de commande **14** des différentes unités de détection **U1**, **U2**, **U3**, Un permettant notamment de détecter et/ou de contrôler les paramètres de mesure, en particulier la température, et de sélectionner le ou les modes de détection des unités de détection parmi un mode statique, un mode dynamique, un mode standard et un mode hybride. Les paramètres de commande de l'unité de commande **14** peuvent y être programmés ou importés.

Un dispositif de détection selon la présente invention prend typiquement la forme d'un boitier rigide comprenant au moins une ouverture permettant un flux d'air vers les éléments de détection. La ou les ouvertures peuvent être pourvue de filtres anti-poussière. Il peut comporter une ou plusieurs interfaces homme-machine tels qu'un dispositif de mise en marche et d'arrêt, un système d'entrée ou de sélection de données ou de programmes préétablis, un dispositif d'affichage d'au moins certaines données telles que le statut d'un cycle de mesure ou un ou plusieurs paramètres de détection, un moyen de connexion apte à transmettre des données, tel qu'une connexion wifi, bluetooth, filaire, internet ou tout équivalent, un système d'alimentation et/ou une batterie.

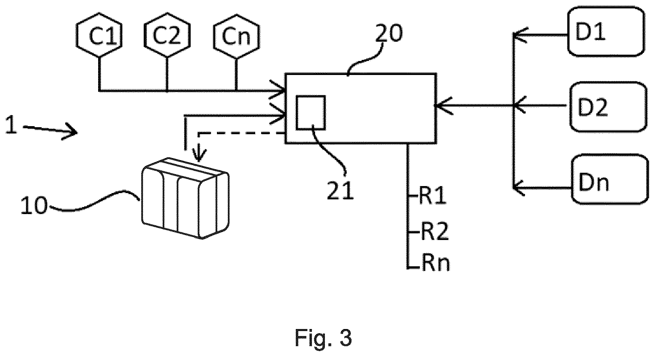

La présente invention couvre en outre un système d'analyse **1** comprenant un ou plusieurs dispositifs de détection **10** tel que décrit ici (figure 3). Le système d'analyse **1** comprend une unité de d'analyse **20** des données collectées par le ou les dispositifs de détection **10**. L'unité de d'analyse des données **20** peut comprendre un module d'intelligence artificielle **21** ou de deep learning ou tout programme apte à l'autoapprentissage. Le module d'intelligence artificielle permet notamment de déduire des profils reçus par le ou les dispositifs de détection **10** la présence d'au moins un biomarqueur gazeux, de préférence plusieurs biomarqueurs gazeux émis par une personne sous surveillance. Il permet en outre de déterminer la variation de concentration du ou des biomarqueurs identifiés au cours du temps. Les variations de concentrations peuvent être déterminées de manière absolue ou de manière relative en référence aux différents composés volatiles détectés. La variation inclut la diminution, l'augmentation, les variations cycliques, les concentrations relatives de plusieurs biomarqueurs.

L'identification d'un ou plusieurs biomarqueurs gazeux peut être obtenu par exemple par comparaison avec une ou plusieurs bases de données préétablies comprenant les profils de biomarqueurs gazeux et des mélanges de tels biomarqueurs gazeux.

Le module d'intelligence artificielle **21** peut être en mesure d'extraire des données collectées de celles relatives à d'éventuels contaminants détectés par le ou les dispositifs de détection **10**. Les profils de contaminants peuvent être par exemple comparés à ceux d'une base de données. Une méthode de déconvolution ou tout autre méthode adaptée à l'identification d'un profil particulier au sein d'une superposition de profils peut être utilisée.

Selon un mode de réalisation, les éventuels contaminants ainsi que la variation de leur concentration le cas échéant, peuvent être identifiés par le module d'intelligence artificielle **21**.

L'unité de d'analyse **20** peut en outre être connectée à un ou plusieurs types de capteurs environnementaux **C1**, **C2**, **Cn** distincts du dispositif de détection **10** ou intégrés au dispositif de détection **10**. De tels capteurs environnementaux incluent des capteurs de température ambiante, des hygromètres, des baromètres, des capteurs d'irradiation lumineuse visible, UV ou infrarouge, des capteurs de bruit tels que des micros, des capteurs de présence et/ou de mouvement. Des capteurs non environnementaux, en particulier des capteurs physiologiques tels que des capteurs de température corporelle, de pression artérielle, d'oxygénation, de glycémie peuvent en outre être connectés à l'unité de traitement de données 20.

L'unité de traitement des données **20** est en mesure de corriger, pondérer, modifier les profils émis par le ou les dispositifs de détection **10** en fonction des données collectées par les capteurs environnementaux et/ou non environnementaux.

Alternativement ou en plus, l'unité de traitement des données **20** comporte des moyens d'accès à des bases de données tierces **D1**, **D2**, **Dn** comportant par exemple des données météorologiques, incluant des données prévisionnelles, des cartographies de pollens, des données de qualité ou de pollution de l'air incluant par exemple les concentrations de microparticules, de nanoparticules, d'ozone, de dioxyde de carbone, de monoxyde de carbone, et d'autres gaz ou éléments en suspension dans l'air.

Selon un mode de réalisation, l'unité de traitement des données est en mesure de corriger, pondérer ou modifier les profils émis par le dispositif de détection **10** en fonction des données collectées dans l'une ou plusieurs de ces bases de données tierces **D1**, **D2**, **Dn.**

Selon un mode de réalisation, l'unité de traitement des données **20** est en mesure de distinguer les composés volatiles issus de pathogènes ou marqueurs d'une pathologies, de ceux qui ne sont pas issus de pathogènes. Il est en effet possible que certains composés volatiles puissent être naturellement émis par une personne en bonne santé, Dans ce cas, le profil spécifique liés à certains composés volatiles peuvent permettre de distinguer entre une situation saine et une situation pathologique.

Selon un mode de réalisation, l'unité de traitement des données **20** est en mesure d'identifier un ou plusieurs biomarqueur gazeux représentatif d'une pathologie ou de l'état de santé du patient.

Selon un mode de réalisation, l'unité de traitement des données **20** est en mesure d'identifier un ou plusieurs biomarqueur gazeux représentatif d'effets secondaires d'un traitement d'une pathologie.

Selon un mode de réalisation, l'unité de traitement des données 20 est en mesure de modéliser l'évolution d'une pathologie ou de ses éventuelles complications sur la base des données collectées par le ou les dispositifs de détection **10**.

L'unité de traitement des données **20** comprend les processeurs, mémoires et logiciels adéquats pour le traitement des données selon la méthode décrite plus loin. L'unité de traitement des données **20** permet de générer, de manière automatique ou sur requête, sur la base des profils reçus du ou des dispositifs de détection **10** et des données reçues par les capteurs environnementaux, non-environnementaux **C1**, **C2**, **Cn** et par les bases de données tierces **D1**, **D2**, **Dn**, le cas échéant, des résultats d'analyses **R1**, **R2, Rn**. L'unité de traitement des données **20** comprend les algorithmes adéquats pour la production de tels résultats d'analyses.

Les résultats d'analyses **R1**, **R2**, **Rn** incluent l'identification de pathogènes responsables des composés volatiles détectés dans l'environnement d'un ou plusieurs patients. Les pathogènes peuvent être identifiés à un stade précoce d'une infection ou d'une pathologie. De préférence, les pathogènes sont ceux d'une maladie respiratoire pour laquelle les prélèvements d'échantillons pour une analyse en laboratoire sont difficiles voir impossibles. Des pathogènes d'autre nature telle que ceux d'une infection non respiratoire ou d'un dysfonctionnement d'un organe peuvent néanmoins être détectés. L'identification de tels pathogènes peut être corrélée à la température corporelle de la personne surveillée, au son d'une éventuelle toux et à d'autres paramètres physiologiques. Une distinction peut être faite entre un pathogène bactérien et un pathogène viral par exemple. Un tel rapport d'analyse peut suggérer un traitement adapté sur la base de l'identification du pathogène. Typiquement, dans le cas d'un pathogène d'origine virale, le traitement peut ne pas suggérer l'utilisation d'antibiotiques. Pour un pathogène de type bactérien, le résultat d'analyse peut être en mesure de suggérer un antibiotique adapté de sorte à éviter l'utilisation infructueuse d'antibiotiques non actifs. Le résultat d'analyse peut comporter le cas échéant une résistance connue du pathogène à certains traitements.

Alternativement ou en plus, les rapports d'analyse peuvent permettre d'identifier les composés volatiles les plus représentatifs d'une pathologie, lesquels doivent alors êtres suivis avec le plus d'attention. Les composés volatiles pertinents ou leurs profils fait l'objet d'une analyse en soi pour permettre de leur attribuer le statut de biomarqueurs gazeux. Tous les composés volatiles détectés ne sont pas représentatifs d'une pathologie. Ils peuvent en outre être émis dans des proportions qui ne témoignent pas d'une pathologie. Ainsi, la détection des composés volatiles selon la présente invention peut être utilisée à des fins de recherche ou d'investigation, outre les applications de diagnostic et de suivi thérapeutique ici décrites

Les résultats d'analyse peuvent inclure des paramètres favorables ou défavorables à la pathologie identifiée, par exemple sur la base des conditions environnementales telles que l'hygrométrie, la température ou la présence de contaminants volatiles.

Les résultats d'analyse peuvent inclure un modèle d'évolution de la pathologie sur la base de mesures continues au moyen du dispositif de détection **10** et le cas échéant des données collectées par des capteurs **C1**, **C2**, **Cn** environnementaux et/ou non environnementaux et des données prévisionnelles ou en temps réel issues de bases de données tierces.

Les résultats d'analyse peuvent comprendre une corrélation entre les données collectées et analysées et un traitement administré au patient. Les profils analysés permettent d'attester d'une rémission d'une pathologie par exemple par diminution de biomarqueurs gazeux spécifiques.

Les résultats d'analyse ne sont pas limités à ceux effectivement décrits ici. Ils peuvent prendre la forme de rapports écrits et/ou numérisés. Les résultats d'analyses peuvent en outre être structurés et distribués en accord avec le destinataire. Les résultats d'analyse peuvent avoir par exemple plusieurs destinataires parmi un médecin traitant, un chercheur, un assureur ou le patient lui-même. Ainsi certains résultats d'une analyse donnée peuvent être transmis seulement à certains destinataires spécifiques. Par exemple, dans le cas d'une surveillance à domicile, des résultats d'analyse concernant l'effet d'un traitement peuvent être transmis au médecin traitant.

La présente invention couvre en outre une méthode de détection d'un ou plusieurs biomarqueurs volatiles au moyen d'au moins un dispositif de détection tel que le dispositif de détection **10** ici décrit. La méthode de détection comprend une étape de disposer au moins un dispositif de détection dans une pièce de sorte à analyser l'air de la pièce. La pièce est de préférence occupée par une ou plusieurs personnes dont la santé est surveillée.

La méthode de détection comprend l'étape d'analyser de manière autonome et/ou automatique un ou plusieurs échantillons d'air de sorte à identifier d'éventuels biomarqueurs gazeux. L'analyse peut être effectuée en continu ou par cycles d'analyse successifs. Un cycle d'analyse comprend un ou plusieurs modes parmi les modes statiques, dynamiques, standards et mixtes décrits plus haut.

La méthode de détection peut comporter une étape de sélectionner un mode de manière automatique dans le cas où les réponses obtenues sont sous un seuil d'intensité prédéterminé.

La méthode de détection peut comprendre une étape ou un cycle de nettoyage de dispositif de détection, de préférence de manière automatique, lorsque les réponses obtenues sont sous un seuil d'intensité prédéterminé. Une étape ou un cycle de nettoyage peut comporter un chauffage d'un ou plusieurs éléments de détection du dispositif de détection à une température supérieure à 200°C ou 300°C ou comprise entre 300°C et 400°C ou une variation cyclique de température, de sorte à détruire d'éventuels contaminants sans endommager les éléments de détection. Le nettoyage peut être associé ou non à un flux d'air permettant d'évacuer d'éventuels contaminants. Alternativement ou en plus, une irradiation UV ou infrarouge d'un ou plusieurs éléments de détection peut être envisagée. L'étape ou le cycle de nettoyage peut être effectué durant une période jugée adéquate pour restaurer la sensibilité et/ou la précision des éléments de détection. Par exemple un cycle thermique d'une minute ou de moins d'une minute peut être envisagé.

La méthode de détection comprend une étape de collecter les signaux de réponse du dispositif de détection de sorte à établir le profil des composés volatiles présents dans la pièce au moment de la mesure. La collecte des signaux s'effectuent durant une période de détection et/ou selon un mode de détection. Une opération de détection peut durer de l'ordre d'une minute ou moins d'une minute. Le profil des composés volatiles peut être établi au moyen d'une unité de traitement des données **13** associée ou intégrée au dispositif de détection. Le profil détecté peut être stocké dans une mémoire du dispositif de détection de manière temporaire ou transmis à une unité d'analyse **20**. Alternativement ou en plus, le profil détecté peut être lu par un dispositif apte à collecter les données stockées dans le dispositif de détection, tel qu'un terminal, un appareil téléphonique ou tout équivalent.

La méthode de détection peut comporter l'itération de plusieurs périodes de détection à des intervalles prédéterminés ou initiés de manière automatique.

La méthode de détection peut comporter une étape de calibration du dispositif de détection en fonction des composants volatiles à identifier. Une telle calibration peut par exemple être effectuée en mesurant des composants volatiles connus à des concentrations et dans des conditions contrôlées, et en enregistrant le signal de réponse.

La présente invention couvre en outre une méthode d'analyse au moyen d'une unité d'analyse des données **20** émises par le ou les dispositifs de détection. La méthode d'analyse peut comprendre la correction, la pondération, le retraitement des profils émis par le ou les dispositifs de détection avec des données externes telles que des données reçues par des capteurs **C1**, **C2**, **Cn** environnementaux ou non environnementaux, ou des bases de données tierces **D1**, **D2**, **Dn**, au moyen d'algorithmes appropriés.

La méthode d'analyse comprend en outre une étape de transmettre des résultats d'analyse **R1**, **R2**, **Rn** en réponse à une requête spécifique ou pour une analyse de routine effectuée de manière automatique. Les résultats d'analyse peuvent être scindés en sous-ensembles transmis chacun à des destinataires différents.

La méthode d'analyse ici décrite peut impliquer plusieurs dispositifs de détection disposés par exemple dans plusieurs chambres d'un hôpital ou chez plusieurs patients en surveillance à domicile. Les données collectées puis analysées peuvent être transmises à un médecin traitant ou tout autre personnel soignant. Les données collectées peuvent différer d'un patient à l'autre. La présente méthode d'analyse permet par exemple de comparer les données successivement reçues par l'unité de d'analyse **20** et de déterminer leur évolution dans le temps. L'analyse peut ainsi permettre de détecter la rémission ou la dégradation d'un patient, les éventuels cofacteurs influençant l'évolution de sa pathologie, les éventuelles résistances microbiennes, les éventuelles contamination nosocomiales, les éventuels épicentres épidémiques. La méthode d'analyse permet de modéliser l'évolution d'une pathologie, l'efficacité d'un traitement, les éventuels effets secondaires d'un traitement.

La présente méthode d'analyse peut en outre comprendre l'étape de détecter et stocker des profils non préalablement identifiés à des fins d'analyse ultérieures.

Pour les fins de la présente description, les composés volatiles organiques désignent tout composé volatile présent dans l'air sous forme gazeuse. Un biomarqueur gazeux désigne un composé volatile organique d'origine métabolique. Un biomarqueur gazeux peut être d'origine fongique, bactérienne, virale, ou provenir de l'activité cellulaire d'un être vivant, en particulier d'un patient. Un biomarqueur est de préférence indicateur ou caractéristique d'une pathologie et permet de préférence d'identifier la pathologie. Un profil de biomarqueurs gazeux est de préférence utilisé pour caractériser une pathologie. Une pathologie désigne ici tout fonctionnement métabolique anormal pouvant être lié à une infection, une contamination, une inflammation ou à un dysfonctionnement. Une pathologie peut désigner une maladie respiratoire d'origine virale ou bactérienne ou autre. Une maladie respiratoire peut être par exemple la tuberculose, la bronchite, la pneumonie, la pleurésie, la bronchopneumonie.

### Numéros de référence employés sur les figures

- 1: Système d'analyse
- 10: Dispositif de détection de VOC
- 10a, 10b, 10c,: Capteurs
- 10n 11a, 11b, 11c,: Cellules
- 11n 12a, 12b, 12c, 12n: thermorégulateurs
- 13: Unité de traitement de données
- 14: Unité de commande
- 20: Unité d'analyse de données
- 21: Module d'intelligence artificielle
- C1, C2, Cn: Capteurs environnementaux ou physiologiques
- D1, D2, Dn: Bases de données tierces
- R1, R2, Rn: Résultats d'analyses

## Revendications

1. Dispositif de détection (10) adapté à l'analyse d'un ou plusieurs échantillons d'air comprenant :
- plusieurs capteurs (10a, 10b, 10c, 10n) de composés volatiles organiques,
- des moyens de détermination et/ou de contrôle d'au moins un paramètre de détection (12a, 12b, 12c, 12n) disposé à proximité ou au niveau de chacun des capteurs (10a, 10b, 10c, 10n),
- une unité de traitement de données (13) apte à collecter les signaux émis par les capteurs au contact du ou des échantillons d'air,
**caractérisé en ce que** les moyens de détermination et/ou de contrôle d'au moins un paramètre de détection sont adaptés à déterminer ou modifier indépendamment les conditions de détection des capteurs et **en ce que** les signaux émis par les capteurs sont traités par l'unité de traitement de données (13) de sorte à générer un profil de composés volatiles organiques.

2. Dispositif selon la revendication 1, les capteurs (10a, 10b, 10c, 10n) étant disposés dans, ou formant, des cellules de détection (11a, 11b, 11c, 11n), chaque cellule de détection comprenant un ou plusieurs capteurs de nature identique ou différente.

3. Dispositif selon la revendication 2, lesdites cellules de détection étant regroupées en une ou plusieurs unités de détection (U1, U2, U3, Un) comprenant chacune plusieurs cellules de détections.

4. Dispositif selon l'une des revendications 1 à 3, lesdits paramètres de détection étant sélectionnés parmi la température, l'hygrométrie et la pression, de préférence la température.

5. Dispositif selon l'une des revendications 1 à 4, dans lequel lesdits capteurs sont sélectionnés parmi des capteurs de type MOS ou de type colorimétrique.

6. Système d'analyse (1) de composés volatiles organiques comprenant :
- au moins un dispositif de détection (10) selon l'une des revendications 1 à 5,
- un ou plusieurs capteurs environnementaux ou non-environnementaux (C1, C2, Cn),
- des moyens d'accès à une ou plusieurs bases de données tierces (D1, D2, Dn) et
- une unité d'analyse (20),
**caractérisée en ce que** l'unité d'analyse (20) est connectée audits au moins un dispositifs de détection, auxdits capteurs (C1, C2, Cn) et audites bases de données tierces (D1, D2, Dn) de sorte à corriger, moduler, pondérer et/ou modifier ledit profile de composés organiques transmis par lesdits au moins un dispositif de détection (10) avec les données reçues par lesdits capteurs (C1, C2, Cn) et bases de données tierces (D1, D2, Dn).

7. Système d'analyse (1) selon la revendication 6, dans lequel l'unité d'analyse (20) comprend un module d'intelligence artificielle (21) apte à identifier au moins un biomarqueur gazeux lié à une pathologie.

8. Méthode de détection de composés volatiles organiques dans un ou plusieurs échantillons d'air au moyen d'un dispositif de détection selon l'une des revendications 1 à 5, comprenant les étapes de :
- mettre les capteurs (10a, 10b, 10c, 10n) de composés volatiles organiques au contact du ou des échantillons d'air,
- acquérir et traiter le signal de réponse des capteurs au moyen de l'unité de traitement des données (13), et
- générer un profil de composés volatiles organiques sur la base du signal des capteurs,
**caractérisée en ce que** les paramètres de détection au niveau de chaque capteur est déterminé et/ou contrôlé indépendamment, lesdits paramètres de détection comprenant un ou plusieurs paramètres parmi la température, l'hygrométrie et la pression.

9. Méthode selon la revendication 8, lesdits capteurs étant regroupés dans plusieurs unités de détection (U1, U2, U3) et l'acquisition du signal de réponse des capteurs étant effectuée indépendamment pour chacune des unités de détection selon l'un des modes parmi un mode statique, un mode dynamique, un mode standard et un mode hybride.

10. Méthode selon la revendication 9, le mode statique étant défini par un paramètre de détection constant et différent pour chaque capteur d'une unité de détection donnée, le mode dynamique étant défini par un paramètre de détection indépendamment variable pour chaque détecteur d'une unité de détection donnée, le mode standard étant défini par un paramètre de détection correspondant aux conditions ambiante pour tous les détecteurs d'une unité de détection donnée, et le mode mixte combinant plusieurs de modes statique, dynamique et standard sur plusieurs unités de détection.

11. Méthode selon la revendication 10, le paramètre de détection étant la température, ladite température étant déterminée entre une température minimale et une température maximale pour chaque capteur d'une unité de détection dans un mode statique, ladite température variant indépendamment entre une température minimale et une température maximale pour chaque capteur d'une unité de détection en mode dynamique, ladite température étant la température ambiante pour tous les capteurs d'une unité de détection en mode standard.

12. Méthode de détection selon l'une des revendications 8 à 11, comprenant en outre une étape d'autonettoyage d'un moins un des capteurs lorsque le signal correspondant est sous un seuil d'intensité prédéterminé.

13. Méthode selon l'une des revendications 9 à 12, comprenant en outre la sélection automatique d'un des modes de détection lorsque le signal de réponse d'un ou plusieurs capteurs d'une unité de détection est inférieur à un seuil d'intensité prédéterminé.

14. Méthode d'analyse de composés volatiles organiques au moyen d'un système d'analyse selon l'une des revendications 6 et 7, comprenant :
- la comparaison des profiles de composés volatile organique reçues par l'unités d'analyse (20) à ceux d'une base de données préétablie, ladite base de données préétablie comprenant des profils de biomarqueurs gazeux,
- l'identification de biomarqueurs gazeux présents parmi les composés volatiles organiques, et
- la production d'un résultat d'analyse (R1, R2, Rn) sur la base des biomarqueurs gazeux identifiés.

15. Méthode d'analyse selon la revendication 14, comprenant en outre une ou plusieurs des étapes de :
- caractériser une pathologie sur la base des biomarqueurs gazeux identifiés,
- comparer les biomarqueurs gazeux au cours du temps et déterminer leur évolution,
- corréler l'évolution des biomarqueurs à un traitement administré comme remède à la pathologie et/ou à d'éventuels contaminants et/ou à d'éventuelles conditions environnementales,
- distinguer une origine virale ou bactérienne de la pathologie identifiée,
- identifier une éventuelle résistance ou d'éventuels effets secondaires liés à un traitement,
- modéliser l'évolution d'une pathologie identifiée, et
- identifier des biomarqueurs clés d'une pathologie donnée.
